(19) Europäisches Patentamt
European Patent Office
Office européen des brevets

(11) Publication number: **0 039 061**
**B1**

(12) **EUROPEAN PATENT SPECIFICATION**

(45) Date of publication of patent specification: **28.12.83**

(51) Int. Cl.[3]: **C 07 C 85/18,**
C 07 C 87/06,
C 07 C 87/62,
C 07 D 295/02,
B 01 J 31/16

(21) Application number: **81103105.3**

(22) Date of filing: **24.04.81**

(54) **Preparation of amines from olefins using certain transition metal catalysts.**

(30) Priority: **28.04.80 US 143985**

(43) Date of publication of application:
**04.11.81 Bulletin 81/44**

(45) Publication of the grant of the patent:
**28.12.83 Bulletin 83/52**

(84) Designated Contracting States:
**BE DE FR GB IT NL**

(56) References cited:
**DE - C - 931 948**
**US - A - 2 387 705**
**US - A - 2 750 417**
**US - A - 4 120 901**

(73) Proprietor: **PENNWALT CORPORATION**
**Pennwalt Building Three Parkway**
**Philadelphia Pennsylvania 19102 (US)**

(72) Inventor: **Gardner, David Milton**
**1525 Green Hill Road**
**Collegeville, PA 19426 (US)**
Inventor: **Clark, Roger Thomas**
**531 Neiman Road**
**Pottstown, PA 19464 (US)**

(74) Representative: **Kraus, Walter, Dr. et al,**
**Patentanwälte Dres. Kraus & Weisert**
**Irmgardstrasse 15**
**D-8000 München 71 (DE)**

**EP 0 039 061 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

Preparation of amines from olefins using certain transition metal catalysts

## BACKGROUND OF THE INVENTION

This invention relates to the preparation of aliphatic and aromatic amines by a catalytic reaction of an olefin with ammonia or a primary or secondary amine. More particularly, this invention relates to the preparation of amines in the presence of a catalyst which is a simple salt, an organometallic compound, or a coordination compound of ruthenium or iron.

Prior-art large volume manufacturing processes for the production of amines involve the reductive amination of alcohols, ethers, ketones, or aldehydes. These processes suffer from the by-product formation of water, which, in the presence of product amines and unreacted starting materials, results in difficult and expensive separation and purifications steps to recover the desired product.

U.S. Patents 2,417,892 and 3,412,158 describe the reaction of ammonia with olefins in the vapor phase over Group VIII metal and metal oxide catalysts to produce a mixture of amines, nitriles, and degradation products. Alkali metals and alkali metal hydrides have also been used in the preparation of amines from olefins in the vapor phase as described in U.S. Patent 2,501,556, but the products here also are mixtures of amines, nitriles, and degradation products. Due primarily to low conversions, poor selectivity to specific amines, and short catalyst life, no known commercial process has been developed based on these technologies.

It has been reported in the literature that coordination compounds of certain transition metals are effective catalysts for a variety of commercially important olefin reactions. These catalysts are thought to function by bonding with both reacting species, and bringing them into close proximity and providing an electronic and steric environment conducive to a desired transformation.

Prior-art attempts have been made to catalyze the addition of ammonia or amines to olefins with coordination compounds of palladium, platinum, and rhodium. These experiments are reported in A. DeRenzi, Gazetta Chemica Italiana *102,* 281 (1972); B. Akermark, J. Orgmet. Chem. *72,* 127 (1974); B. Akermark, Tetrahedron Letters *15,* 1363 (1974); A. Panunzi, JACS *91,* 3879 (1969); E. Benedetti, Gazetta Chemica Italiana *102,* 744 (1972); and D. Hollings, J. Orgmet. Chem. *54,* 399 (1973). With these coordination compounds of palladium and platinum, organometallic intermediates are obtained that can be hydrolyzed to alkyl amines in high yields. Since the hydrolysis is irreversible, the reaction is stoichiometric rather than catalytic.

U.S. Patent 3,758,586 discloses that certain coordination compounds of rohodium, secondary amines and ethylene react catalytically to give free tertiary amines. It is also disclosed that ammonia and primary amines are ineffective in this reaction, and that the reaction is operative only with ethylene.

German Patent 931,948 discloses a process for the production of secondary or tertiary amines, which comprises reacting an olefin with carbonmonoxide, water and a neutralized primary or secondary amine in the presence of a catalyst, preferably iron carbonyl.

No prior art was found that suggests that either ruthenium or iron compounds could be used to catalyze the simple addition reaction of an olefin and ammonia or amine. These ruthenium and iron compounds are commercially attractive because they are less expensive than platinum, palladium, or rhodium compounds.

This invention is directed to a process for the production of aliphatic and aromatic amines which comprises reacting an olefin with either ammonia, a primary amine or a secondary amine in the presence of a catalyst dissolved in a liquid phase solvent at a temperature of 100° to 250°C and at a pressure from atmospheric to 826.01 bar (12,000 psig), said catalyst being selected from the class consisting of

i) a ruthenium or iron salt,

ii) a coordination compound of ruthenium or iron, and

iii) an organo-metallic compound of ruthenium or iron,

whereby an N—H bond is added across the double bond of the olefin.

A series of iron catalysts have now been discovered that catalyze the addition of primary amines, secondary amines, or ammonia to olefins. In addition, it has been discovered that simple salts of ruthenium as well as certain coordination compounds of ruthenium are also catalysts for this reaction.

The present invention can be described by the equation:

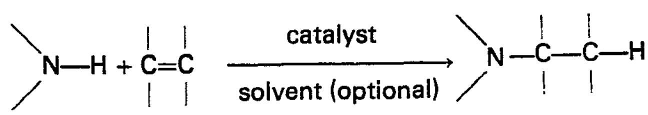


which indicates that an N—H bond adds across the double bond of an olefin.

Suitable amines include ammonia, dimethylamine, diethylamine, piperidine, and other such primary and secondary amines.

The olefins used have been alpha-olefins including ethylene, propylene, and isobutylene. Other homologs may be used.

Solvents used in this invention can be either the reactants or products, or can be solvents chemically inert to reactants and products that are capable of dissolving substantial amounts of the particular catalyst. Typical examples are: saturated aliphatic hydrocarbons, such as hexane, heptane, cyclohexane; aromatic hydrocarbons, such as toluene, xylene and benzene; ethers such as tetrahydrofuran, 1,4-dioxane, ethylene glycol monomethylether; saturated aliphatic alcohols, such as methanol and ethanol; and the reactant or product amines, such as diethylamine, piperidine, and triethylamine.

Temperatures in the range of 100° to 235°C are useful for carrying out this invention. The preferred temperature range is 100° to 190°C.

Pressures in the range of atmospheric to 826.01 bar (12,000 psig) are useful for carrying out this invention. Pressures in the range of 14.76 to 104.14 bar (200 psig to 1500 psig) are preferred.

The proportion of olefin, amine, and catalyst are not critical and any amounts of these three components which are brought together under the conditions of this invention will produce the desired product amine(s).

The catalyst is any of three types of ruthenium or iron compounds. The first type is a simple salt of either ruthenium or iron. The second type is a coordination compound of either ruthenium or iron. The third type is an organometallic compound of either ruthenium or iron.

The preferred ruthenium and iron salts are salts of strong mineral acids or organic acids. Specific examples of such salts include the chloride, bromide, iodide, nitrate, sulfate, phosphate, formate, and acetate salts and their hydrates.

The preferred coordination compounds of ruthenium are:

$RuCl_2$(triphenylphosphine)$_3$;
Ru(cyclopentadienyl)$_2$;
Ru(2,4-pentanedionate)$_3$;
Ru(dimethylglyoxime)$_2$Cl;
Ru(2-cyanopyridine)$_2$Cl;
Ru(triphenylarsine)$_2Cl_3$;
Ru(N,N-ethylene-bis-salicylideneamine)$Cl_3$;
Ru(8-hydroxyquinoline)$_2$Cl;
Ru(2-hydroxyquinoline)$_2$Cl;
Ru(dimethylglyoxime)$_4Cl_2$;
Ru(2,2′-bipyridine)$_2$Cl;
$K_4Ru(CN)_6 \cdot 3H_2O$;
$[Ru(NH_3)_4OH\ Cl]Cl \cdot 2H_2O$;
$Ru(NO)(NO_3)_3$.

The preferred coordination compounds of iron are:

$Fe(CO)_5$
$Fe_2(CO)_9$
Fe(butadiene)$(CO)_3$
HgCl(cyclopentadienyl)$_2$Fe
$H_2Fe(CO)_4$
Fe(cyclohexadienyl)$(CO)_3$
$FeHg(CO)_4$
$Fe(CO)_4[(C_6H_5)_3P]$
$Fe(CO)_4[(C_6H_5O)_3P]$

The amount of catalyst employed in this process is from about 0.1 to 50 mole% of the total moles" reactants, i.e., olefin and ammonia or amine. Preferably the amount of catalyst is kept as low as possible and yet sufficient to provide good yields and good kinetics. Normally 0.5—10 mole% is sufficient to accomplish these objectives.

The process of this invention is nonlimiting. It may be carried out on a batch basis, or it is equally adaptable to a continuous basis by employing known means for introducing the reactants, catalyst, and optional solvent to the reactions zone and for withdrawing the products and recovering starting materials therefrom. As indicated by the low molar percentage of catalyst to reactants, the reaction is truly catalytic rather than stoichiometric in nature.

## Examples

The following examples illustrate various embodiments of this invention. Parts and percentages are by weight, temperatures are in degrees Centigrade, and pressures are in pounds per square inch gauge unless otherwise specified. Conversions and yields given in these examples are based on the isolated liquid products. Major products were identified by gas chromatographic and mass spectroscopic analytical methods, and when necessary, were confirmed by infrared spectroscopy.

Those minor products which are not identified are assumed to have the same gas chromatographic response factor as the major amine product.

In all the test runs it has been observed that no products are formed in either the absence of catalyst or in the presence of an ineffective catalyst.

Example 1

A mixture of 89 parts of tetrahydrofuran, 5.6 parts of dimethylamine and 0.315 g of ruthenium trichloride hydrate were placed in a 300 $cm^3$ stainless steel autoclave. The autoclave was pressured with 1.70 bar (10 psig) of hydrogen and 14.76 bar (200 psig) of ethylene. The mixture was heated in stages at 150°C (1 h), 170°C (1 h), and 190°C (5 h). A total of 0.16 parts of dimethylethylamine and 3.3 parts of diethylmethyl amines were obtained corresponding to a conversion of 35% and a combined yield of 92%.

Example 2

A mixture of 89 parts of tetrahydrofuran, 5.6 parts of dimethylamine and 0.289 parts of $Ru(cyclopentadienyl)_2$ were placed in a 300 ml stainless steel autoclave. The autoclave was pressured with 14.76 bar (200 psig) of ethylene. The mixture was heated in stages at 150°C (1 h), 170°C (1 h), and 190°C (5 h). A total of 1.3 parts of dimethylethylamine were obtained corresponding to a conversion of 1% and a·yield of 99% (based on dimethylamine).

Example 3

A mixture of 104 parts of 1,4-dioxane, 5.6 parts of dimethylamine and 0.363 parts of $[Ru(NH_3)_4(OH)Cl] \cdot 2H_2O$ were placed in a 300 ml stainless steel autoclave. The autoclave was pressured with 14.76 bar (200 psig) of ethylene. The mixture was heated in stages at 150°C (1 h), 170°C (1 h), and 190°C (5 h). A total of 0.12 parts of dimethylethylamine and 2.1 parts of diethylmethylamine were obtained corresponding to a conversion of 21% and a combined yield of 99% (based on dimethylamine).

Example 4

A mixture of 104 parts of 1,4-dioxane and 0.315 parts of ruthenium trichloride hydrate were placed in a 300 $cm^3$ stainless steel autoclave. The autoclave was pressured with 14.76 bar (200 psig) of anhydrous ammonia and 14.76 bar (200 psig) of ethylene. The mixture was heated in stages at 150°C (1 h), and 170°C (1 h), and 190°C (5 h). The resulting reaction mixture was analyzed by gas liquid chromatography and was found to contain monoethylamine, diethylamine, and triethylamine.

Example 5

A mixture of 18.3 parts of anhydrous diethylamine and 2.0 parts of $Fe(CO)_5$ was placed in a 300 ml stainless steel autoclave. The autoclave was pressured with 21.64 bar (300 psig) of ethylene. The mixture was heated in stages at 150°C (1 h) and 170°C (5 h). A total of 6.6 parts of triethylamine and 0.4 parts of monoethylamine were obtained corresponding to a conversion of 28.2% and a combined yield of 100% (based on diethylamine).

Example 6

A mixture of 100 parts of toluene, 9.1 parts of diethylamine and 0.97 parts $Fe(butadiene)(CO)_3$ were placed in a 300 $cm^3$ stainless steel autoclave. The autoclave was purged with ethylene and pressured with ethylene to 14.76 bar (200 psig). The mixture was heated in stages at 150°C (1 h), 170°C (1 h), and 190°C (5 h). A total of 1.6 parts of triethylamine were obtained corresponding to a conversion of 12.8% and a yield of 100% (based on diethylamine).

Example 7

A mixture of 100 parts of 1,4-dioxane, 9.1 parts of diethylamine and 0.98 parts of $Fe(CO)_5$ were placed in a 300 $cm^3$ stainless steel autoclave. The autoclave was purged with ethylene and pressurized with ethylene to 14.76 bar (200 psig). The mixture was heated in stages at 150°C (1 h), 170°C (1 h), and 190°C (5 h). A total of 1.8 parts of triethylamines were obtained corresponding to a conversion of 14.1% and a yield of 93% (based on diethylamine).

Example 8

A mixture of 55 parts of ammonia and 0.98 parts of $Fe(CO)_5$ were placed in a 300 $cm^3$ stainless steel autoclave. The autoclave was pressured with 14.76 bar (200 psig) of ethylene. The mixture was heated at 120°C for 5 hrs. The autoclave was chilled with a dry ice/acetone bath opened to the atmosphere and 100 parts of toluene added. After warming to room temperature with the resultant evaporation of excess ammonia, the mixture was analyzed by gas liquid chromatography and was found to contain monoethylamine, diethylamine and triethylamine.

Example 9

A mixture of 10 parts of ammonia, 1.96 parts of $Fe(CO)_5$, 2.02 parts of tri-n-butylphosphine and 14 parts of isobutylene were placed in a 300 $cm^3$ stainless steel autoclave. The autoclave was sealed and heated to 170°C for 5 hrs. The autoclave was chilled with a dry ice/acetone bath, opened to the atmosphere and 50 parts of toluene added. After warming to room temperature with the resultant evaporation of excess ammonia and isobutylene, the mixture was analyzed by gas liquid chromatography and was found to contain isobutylamine.

Example 10

A mixture of 21.3 parts of piperidine, 1.96 parts of $Fe(CO)_5$ and 0.27 parts of mercuric chloride were placed in a 300 $cm^3$ stainless steel autoclave. The autoclave was purged with ethylene and pressurized with ethylene to 21.64 bar (300 psig). The mixture was heated in stages at 150°C (1 h) and 170°C (5 h). A total of 2.1 parts of N-ethylpiperidine were obtained corresponding to a conversion of 8% and a yield of 99% (based on piperidine).

Example 11

A mixture of 18.3 parts of anhydrous diethylamine, 2.0 parts of $Fe(CO)_5$ and 3.1 parts of triphenylphosphite were placed in a 300 ml stainless steel autoclave. The autoclave was pressured with 21.64 bar (300 psig) of ethylene. The mixture was heated in stages at 150°C (1 h) and 170°C (5 h). A total of 10.5 parts of triethylamine and 0.6 parts of monoethylamine were obtained corresponding to a conversion of 47.4% and a combined yield of 98.4 (based on diethylamine).

Example 12

A mixture of 23.3 parts of anhydrous aniline, 2.0 parts of $Fe(CO)_5$ and 3.1 parts of triphenylphosphite were placed in a 300-ml stainless steel autoclave. The autoclave was pressured with 21.64 bar (300 psig) of ethylene. The mixture was heated in stages at 130°C (1 h), 140°C (1 h) and 150°C (5 h). A total of 3 parts of N-ethyl aniline, 1 part 2-methylquinoline, 1 part diethyl aniline, 0.5 parts 2-(1-butenyl)aniline and 0.25 parts of N-ethyl toludine were obtained corresponding to a conversion of 17.3% and a combined yield of 100% (based on aniline).

**Claims**

1. A process for the production of aliphatic and aromatic amines which comprises reacting an olefin with either ammonia, a primary amine, or a secondary amine in the presence of a catalyst dissolved in a liquid phase solvent at a temperature of 100°C to 250°C, and at a pressure from atmospheric to 826.01 bar (12000 psig), said catalyst being selected from the class consisting of
   i) a ruthenium or iron salt,
   ii) a coordination compound of ruthenium or iron, and
   iii) an organo-metallic compound of ruthenium or iron,
whereby an N—H bond is added across the double bond of the olefin.

2. The process of Claim 1 wherein the temperature is 110°—190°C and the pressure is 14.76—104.14 bar (200—1500 psig).

3. The process of Claim 1 wherein the solvent must be capable of dissolving substantial amounts of the catalyst and is selected from the class consisting of
a) the reactants or products and
b) a solvent chemically inert to the reactants and products selected from
   1) a saturated aliphatic hydrocarbon,
   2) an aromatic hydrocarbon,
   3) an ether, a saturated aliphatic alcohol, or
   4) an amine.

4. The process of Claim 1 wherein the ruthenium or iron salt is a salt or a salt hydrate of a strong mineral acid or an organic acid.

5. The process of Claim 1 wherein the coordination complex of ruthenium is selected from the group consisting of:

$RuCl_2$ (triphenylphosphine)$_3$,
Ru (cyclopentadienyl)$_2$,
Ru (2,4-pentanedionate),
Ru (dimethylglyoxime)$_2$Cl,
Ru (2-cyanopyridine)$_2$Cl,
Ru (triphenylarsine)$_2Cl_3$,
Ru (N,N-ethylene-bis-salicylideneamine)$Cl_3$,
Ru (8-hydroxyquinoline)$_2$Cl,
Ru (2-hydroxyquinoline)$_2$Cl,
Ru (dimethylglyoxime)$_4Cl_2$,

Ru (2,2'-bipyridine)$_2$ Cl,
$K_4Ru(CN)_6 \cdot 3H_2O$,
Ru $(NH_3)_4$ OH·$Cl_2$ $2H_2O$, and
Ru (NO) $(NO_3)_3$.

6. The process of Claim 1 wherein the coordination compound of iron is selected from the group consisting of:

$Fe(CO)_5$,
$Fe_2(CO)_9$,
Fe (butadiene)$(CO)_3$,
HgCl(cyclopentadienyl)$_2$Fe,
$H_2Fe(CO)_4$,
Fe(cyclohexadienyl)$(CO)_3$,
FeHg$(CO)_4$,
$Fe(CO)_4$ (triphenylphosphine), and
$Fe(CO)_4$ (triphenylphosphite).

**Revendications**

1. Procédé de production d'amines aliphatiques et aromatiques, qui consiste à faire réagir une oléfine avec l'ammoniac, une amine primaire ou une amine secondaire en présence d'un catalyseur dissous dans un solvant en phase liquide à une température de 100 à 250°C et à une pression allant de la pression atmosphérique à 826,01 bars (pression manométrique de 12 000 lb/in$^2$), ledit catalyseur étant choisi dans la classe comprenant
i) un sel de ruthénium ou de fer,
ii) un composé de coordination du ruthénium ou du fer, et
iii) un composé organométallique du ruthénium ou du fer,
une liaison N—H étant ajoutée d'un côté de la double liaison de l'oléfine.

2. Procédé suivant la revendication 1, dans lequel la température est de 110—190°C et la pression est de 14,76—104,14 bars (200—1500 lb/in$^2$ au manomètre).

3. Procédé suivant la revendication 1, dans lequel le solvant doit être capable de dissoudre des quantités importantes du catalyseur et est choisi dans la classe comprenant
a) les corps réactionnels ou les produits et
b) un solvant chimiquement inerte vis-à-vis des corps réactionnels et des produits, choisi entre
1) un hydrocarbure aliphatique saturé,
2) un hydrocarbure aromatique,
3) un éther, un alcool aliphatique saturé, ou
4) une amine.

4. Procédé suivant la revendication 1, dans lequel le sel de ruthénium ou de fer est un sel ou un sel hydraté d'un acide minéral fort ou d'un acide organique.

5. Procédé suivant la revendication 1, dans lequel le complexe de coordination du ruthénium est choisi dans le groupe comprenant:

$RuCl_2$ (triphénylphosphine)$_3$,
Ru (cyclopentadiényle)$_2$,
Ru (2,4-pentanedionate),
Ru (diméthylglyoxime)$_2$Cl,
Ru (2-cyanopyridine)$_2$Cl,
Ru (triphénylarsine)$_2Cl_3$,
Ru (N,N-éthylène-bis-salicylidène-amine)$Cl_3$,
Ru (8-hydroxyquinoléine)$_2$Cl,
Ru (2-hydroxyquinoléine)$_2$Cl,
Ru (diméthylglyoxime)$_4Cl_2$,
Ru (2,2'-bipyridine)$_2$Cl,
$K_4Ru(CN)_6 \cdot 3H_2O$,
Ru $(NH_3)_4OH \cdot Cl_2$ $2H_2O$, et
Ru (NO)$(NO_3)_3$.

6. Procédé suivant la revendication 1, dans lequel le composé de coordination du fer est choisi dans le groupe comprenant:

$Fe(CO)_5$,
$Fe_2(CO)_9$,
Fe (butadiène)$(CO)_3$,

HgCl(cyclopentadiènyle)$_2$Fe,
$H_2Fe(CO)_4$,
Fe(cyclohexadiényle)$(CO)_3$,
$FeHg(CO)_4$,
$Fe(CO)_4$ (triphénylphosphine), et
$Fe(CO)_4$ (triphénylphosphite).

**Patentansprüche**

1. Verfahren zur Herstellung von aliphatischen und aromatischen Aminen, dadurch gekennzeichnet, daß man ein Olefin entweder mit Ammoniak, einem primären Amin oder einem sekundären Amin in Gegenwart eines Katalysators, gelöst in einem Lösungsmittel in flüssiger Phase bei einer Temperatur von 100°C bis 250°C und einem Druck von Atmosphärendruck bis 826,01 bar (12000 psig), umsetzt, wobei der Katalysator aus der Klasse, bestehend aus
i) einem Ruthenium- oder Eisensalz,
ii) einer Koordinationsverbindung von Ruthenium oder Eisen und
iii) einer organometallischen Verbindung von Ruthenium oder Eisen,
ausgewählt wird, wodurch eine N—H-Bindung über die Doppelbindung des Olefins angefügt wird.

2. Verfahren nach Anspruch 1, dadurch gekennzeichnet, daß die Temperatur 110°C bis 190°C ist und daß der Druck 14,76 bis 104,14 bar (200 bis 1500 psig) ist.

3. Verfahren nach Anspruch 1, dadurch gekennzeichnet, daß das Lösungsmittel dazu imstande sein muß, erhebliche Mengen des Katalysators aufzulösen, und aus der Klasse, bestehend aus
a) den Ausgangsstoffen oder Produkten und
b) einem Lösungsmittel, das gegenüber den Ausgangsstoffen und Produkten inert ist, ausgewählt aus
1) einem gesättigten aliphatischen Kohlenwasserstoff,
2) einem aromatischen Kohlenwasserstoff,
3) einem Ether, einem gesättigten aliphatischen Alkohol oder
4) einem Amin,
ausgewählt wird.

4. Verfahren nach Anspruch 1, dadurch gekennzeichnet, daß das Ruthenium- oder Eisensalz ein Salz oder ein Salzhydrat einer starken Mineralsäure oder einer organischen Säure ist.

5. Verfahren nach Anspruch 1, dadurch gekennzeichnet, daß der Koordinationskomplex von Ruthenium aus der Gruppe, bestehend aus

$RuCl_2$-(triphenylphosphin)$_3$,
Ru-(cyclopentadienyl)$_2$,
Ru-(2,4-pentandionat),
Ru-(dimethylglyoxim)$_2$Cl,
Ru-(2-cyanopyridin)$_2$Cl,
Ru-(triphenylarsin)$_2Cl_3$,
Ru-(N,N-ethylen-bis-salicylidenamin)-$Cl_3$,
Ru-(8-hydroxychinolin)$_2$Cl,
Ru-(2-hydroxychinolin)$_2$Cl,
Ru-(dimethylglyoxim)$_4Cl_2$,
Ru-(2,2′-bipyridin)$_2$Cl,
$K_4Ru(CN)_6 \cdot 3H_2O$,
$Ru(NH_3)_4OH \cdot Cl_2 \cdot 2H_2O$ und
$Ru(NO)(NO_3)_3$,

ausgewählt ist.

6. Verfahren nach Anspruch 1, dadurch gekennzeichnet, daß die Koordinationsverbindung von Eisen aus der Gruppe, bestehend aus

$Fe(CO)_5$,
$Fe_2(CO)_9$,
Fe-(butadien)$(CO)_3$,
HgCl-(cyclopentadienyl)$_2$Fe,
$H_2Fe(CO)_4$,
Fe-(cyclohexadienyl)$(CO)_3$,
$FeHg(CO)_4$,
$Fe(CO)_4$(triphenylphosphin) und
$Fe(CO)_4$(triphenylphosphit),

ausgewählt ist.